# EUROPEAN PATENT SPECIFICATION

(11) **EP 2 060 276 B1**
(45) Date of publication and mention of the grant of the patent: **25.04.2012**
(21) Application number: 08168887.1
(22) Date of filing: 12.11.2008
(51) Int. Cl.: A61L 2/18, A61L 2/24

(54) **Endoscope reprocessor with multiple adjustable pumps**
Endoskopreinigungsvorrichtung mit mehreren, regelbaren Pumpen
Dispositif de nettoyage d'endoscopes avec plusieurs pompes reglables

(30) Priority: 13.11.2007 NL 2001002
(43) Date of publication of application: 20.05.2009
(73) Proprietor: Wassenburg & Co. B.V., 6669 NA Dodewaard (NL)
(72) Inventor: Wassenburg, Ronald, 6669 DS Dodewaard (NL)
(74) Representative: Eveleens Maarse, Pieter

(56) References cited:
- EP-A- 0 271 157
- EP-A- 1 757 313
- US-A1- 2006 051 285
- US-A1- 2007 015 968
- US-A1- 2007 102 045

## Description

The present invention relates to a device for cleaning endoscopes comprising a group of several endoscope channels, comprising: positioning means for placing the endoscope to be cleaned, a reservoir for cleaning liquid, supply means for supplying cleaning liquid from the reservoir to the endoscope channels, forming part of the group, of an endoscope to be cleaned placed in the positioning means, discharge means for discharging to the reservoir the cleaning liquid leaving the endoscope for cleaning, wherein for each of the endoscope channels to be cleaned and forming part of the group, the supply means comprise a channel which leads from the reservoir to a connecting piece adapted for connection to the endoscope channels, and a pump is placed in each of the channels, and wherein the pumps are all of a type which, when non-operational, forms a closure of the channel.

Such a device is known from EP-A- 1 757 313.

The majority of endoscopes are provided with several channels having a mutually differing minimal passage area. When a pressure is applied to the relevant channels, the flow rate flowing through an endoscope channel will depend on the pressure and the minimal passage area of the relevant endoscope channel. In the above stated prior art the same pressure will be applied to each of the endoscope channels, so that the flow rate depends on the minimum passage area of the relevant endoscope channel. For an optimum flushing or cleaning effect there is an optimum flow rate for each channel. Because the flow rate in each endoscope depends directly on the pressure and the passage area, an optimum flow rate can be realized for only one of the endoscope channels. Such a device is disclosed in US-A-2007/0102045.

As the results of the cleaning may vary, mainly due to the possibility of obstructions in the tiny channels, which could lead to heath hazards, there is a wish to check whether the cleaning of the channels was performed satisfactorily.

The aim of the invention is to provide a cleaning device allowing to check the quality of the cleaning action.

This aim is reached by a device according to claim 1.

It is noted here that the term continuous in the sense of the present measure is also understood to mean a digital and thus necessarily discrete regulation with a sufficient number of steps, for instance at least sixteen.

The invention also relates to a method for cleaning an endoscope, comprising the steps of positioning the endoscope for cleaning in a cleaning device using positioning means, connecting feed channels connected to pumps onto the channels of the endoscope to be cleaned, flushing the endoscope channels at a first pump power, and flushing the endoscope channels at a second pump power differing from the first, wherein the pressure-flow characteristic of the endoscope channel is measured.

Yet another embodiment provides the measure that the device comprises a memory for storing the measured pressure-flow characteristic, and that the pressure-flow characteristic for each of the channels is stored with an identification of the endoscope. This enables a comparison between present and past measured characteristics so that the development through time of the situation of the endoscope channels can be obtained. According to this embodiment the pressure-flow characteristic for each of the channels is moreover stored with an identification of the endoscope.

This latter measure provides the option, after a subsequent cleaning of the same endoscope, of once again measuring the pressure-flow characteristic and comparing the measured pressure-flow characteristic to the stored characteristic.

The use of a regulable motor not only provides the possibility of precise adjustment of the relevant flow rate, but also the possibility that the pumps can operate successively at different flow rates. This can enhance the cleaning process, for instance by removing coarse contaminants with a high flow rate and removing small contaminants with a low flow rate. For this purpose the invention likewise preferably relates to such a method wherein the second power is lower than the first power.

According to a particular embodiment, after flushing of an endoscope channel at a high pump power, a detection takes place as to whether the endoscope channel is blocked, and said endoscope channel is flushed at a low power only if it is found that the channel is not blocked. An efficient procedure for cleaning the endoscope is hereby obtained. This is because the cleaning process is completed when it is found that the blockage has been removed, while relevant measures, such as flushing at an increased pressure or generating a warning signal, can be taken when it is found that the blockage has not been removed.

A specific preferred embodiment provides the measure that, after flushing at the second power, the endoscope channels are flushed at a third power greater than the second power. A final check for blockage, constriction and connection of the channels can hereby be performed. It is after all possible for a blockage to still occur during flushing at a low power, for instance as a result of temperature differences. This can only be detected by this final flushing at a great power.

The inventors have found that the best cleaning results are obtained when the control device is adapted to cause the pumps to operate initially at a high flow rate and subsequently at a low flow rate. This is because the coarse contaminants and possible blockages are hereby initially removed, while a somewhat more precise cleaning resulting in disinfection can then be obtained when flushing takes place at a lower flow rate, this latter preferably for a longer period of time.

It is sometimes the case that connecting pieces are suitable for connection to endoscopes which are of substantially the same type but which have different numbers of channels. In this latter case it can occur that a connection of the connecting piece does not fit onto an endoscope channel, for instance when an endoscope with a smaller number of channels is connected. In order to prevent an excessively large flow being pumped to no purpose in such a situation, which results in unnecessary wear, a preferred embodiment of the invention provides the measure that the pumps are adapted to generate a flow rate lower than a predetermined flow rate when a connection is not connected to an endoscope channel.

As already stated in the preamble, it is important in preventing contamination by backflow of cleaning liquid from the endoscope that the pumps form a closure when non-operational. Many types of displacement pump in particular meet this criterion, while they are moreover suitable for the pumping of gas, and are thereby self-priming so that they also operate during startup of the cleaning device. Another advantage of displacement pumps is that, as a result of their ability to pump gas, they can be used to empty, by displacement, the conduits connected thereto so that these pumps can be used to empty the whole device.

A further embodiment provides the measure that the pumps are formed by peristaltic pumps. Pumps of this type have the advantage that they can be easily cleaned since only the channel manufactured from flexible material comes into contact with the cleaning liquid.

An endoscope usually comprises a single endoscope channel which has a larger section than the other endoscope channels. It is therefore structurally attractive to provide for this endoscope channel with a larger section a large pump which is adapted to generate a higher flow rate than any of the other pumps, and that this large pump is adapted for connection to an endoscope channel with a larger passage area than the endoscope channels forming part of the group. This is because this channel requires a higher flow rate for an adequate cleaning.

It is noted here that there are endoscopes wherein channels are mutually connected inside the endoscope. There are thus endoscopes wherein the channel with a large section is connected inside the endoscope to one or more channels of the above stated group which thus have a smaller section. The above elucidated measure of providing such a 'large' pump can advantageously be used in such internal endoscopes. The 'large' pump is here connected to the channel with a large section, and one or more channels forming part of the group and connected internally to the channel with a large passage area are connected to normal pumps provided with measuring equipment. It hereby becomes possible for the pump with the high flow rate to supply sufficient flow for the purpose of 'filling' the combined channel, so that the other pump provided with detecting and measuring means can perform the detection function.

According to a further development of the above stated preferred embodiment, the large pump is connected by means of a non-return valve to the connection of the endoscope, and there is connected between the connection of the endoscope and the non-return valve a pump of the type which is adapted for connection to channels forming part of the group. Such a 'large' pump will generally not be of the type which forms a closure when non-operational, so that it is necessary to arrange a non-return valve in order to prevent backflow of the cleaning liquid. The 'parallel connection' to a normal pump and the associated measuring equipment makes it possible, also in the case of this channel with a large section, to apply detection of blockage, correct fitting and measuring of characteristics.

This pump with a higher flow rate can advantageously also be used to clean the endoscope on the outside. A particular preferred embodiment therefore provides the measure that the pump with a higher flow rate is not only adapted for connection to an endoscope channel but also to a sprayer for spraying the endoscope placed in the positioning means.

The present invention will be elucidated hereinbelow with reference to the accompanying figures, in which
Figure 1 shows a schematic representation of a cleaning device according to the invention; and
Figure 2 is a schematic detail representation of a variant of the invention shown in figure 1.

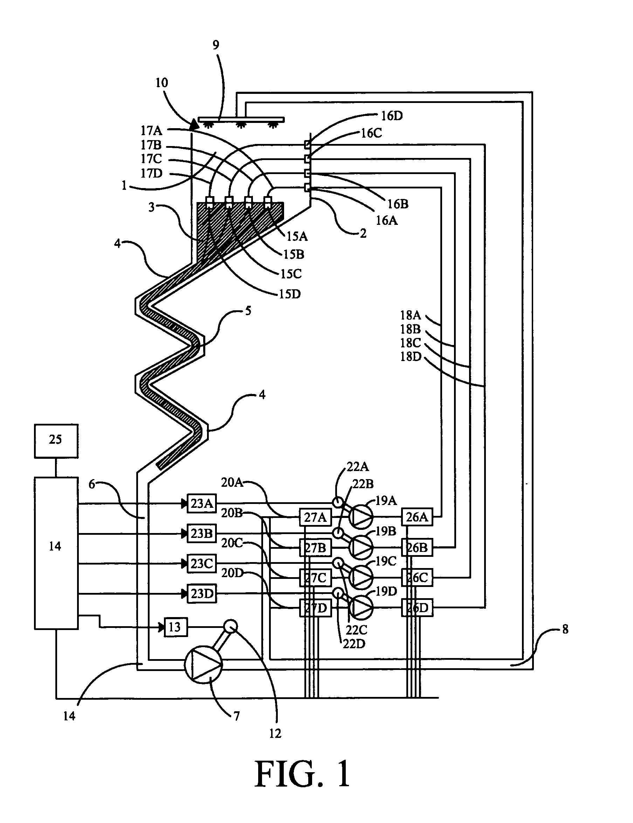

The device shown in figure 1 comprises a space 1 enclosed by a vessel 2 for containing an endoscope 3 for cleaning. Connected to space 1 is a helical channel 4 in which the long flexible part 5 of the endoscope for cleaning can be placed. This helical channel 4 transposes into a discharge conduit 6. Vessel 2 together with helical channel 4 functions here as supply vessel for the cleaning liquid used in the cleaning. It is otherwise also possible to provide a separate supply vessel for the cleaning liquid. Discharge conduit 6 leads to the suction side of a pump 7, the pressure side of which is connected to a feed conduit 8 which leads to a sprayer 9 provided with nozzles 10 for the purpose of spraying the outer side of the endoscope 3 placed in space 1. Pump 7 is coupled to an electric motor 12 which can be powered and controlled by means of a controllable power supply circuit 13. A control device 14 is arranged for this purpose which is adapted to control, among other parts, power supply circuit 13.

Endoscope 4 for cleaning comprises in the present example four channels, each connected to a connection 15A, 15B, 15C and 15D which are received in the actual endoscope body 3 and which during normal use of the endoscope are used for the connection of auxiliary equipment such as cameras or biopsy means or gas and liquid feed or extraction systems. The other end of the channels of the endoscope debouches freely into the space.

Arranged in a wall of vessel 2 are four connections 16A, 16B, 16C and 16D which can each be connected by means of a hose 17A, 17B, 17C and 17D provided with appropriate couplings to associated connections 15A, 15B, 15C and 15D in the endoscope. It will be apparent that the invention is not limited to this number of four endoscope channels; the invention rather comprises devices suitable for cleaning other numbers of endoscope channels, such as 5, 6 or 7, or a small number such as 2 or 3. Connections 16A, 16B, 16C and 16D are each connected by four respective feed conduits 18A, 18B, 18C and 18D to the pressure side of a pump 19A, 19B, 19C and 19D respectively. The suction side of each of these pumps 19A, 19B, 19C and 19D is connected by means of respective feed conduits 20A, 20B, 20C and 20D to feed conduit 8. Pumps 19A, 19B, 19C and 19D are preferably formed by displacement pumps, and in particular by peristaltic pumps. Other types of pump are however not precluded. Each of the pumps 19A, 19B, 19C and 19D is connected for mechanical driving to a respective electric motor 22A, 22B, 22C and 22D. These electric motors can be formed by direct current motors, although they can also be formed by rotary field motors such as synchronous or asynchronous motors. Instead of electric motors use can be made of motors driven by compressed air which must be readily regulable. Electric motors 22A, 22B, 22C and 22D are each connected for power supply to a respective controllable power supply circuit 23A, 23B, 23C and 23D. These power supply circuits 23A, 23B, 23C and 23D are each connected for control purposes to control circuit 14. The type of power supply circuit is here adapted to the type of electric motor applied. Because the control circuit preferably takes a digital form, the power supply circuits take a digitally controllable form.

Using the configuration described thus far it is possible to clean endoscopes better than according to the prior art because each of the electric motors 22A, 22B, 22C and 22D is individually controllable. Owing to this controllability the power and rotation speed of electric motors 22A, 22B, 22C and 22D can be individually regulated, and thereby the power and rotation speed of pumps 19A, 19B, 19C and 19D, and as a result thereof the flow rate and - also subject to the resistance encountered by the pumped-up liquid - also the pressure generated by the pumps. A great freedom of control is hereby obtained so that the power of each of the electric motors and pumps can be adapted to the passage area of the relevant endoscope channels of the relevant endoscope 3 which are connected via conduits 18A, 18B, 18C and 18D. As a result of their differing functions, these endoscope channels do after all usually have differing passage areas. This controllability moreover provides the option of generating not only mutually differing flow rates and pressures but also pressures and flow rates which differ through time. This latter is advantageous in optimizing the cleaning process, such as the initial removal of coarse contaminants with high pressure and high flow rate and subsequently disinfecting with a low flow rate and a low pressure, although for instance for a longer period of time. Use can for instance be made for this purpose of a memory which is connected to control circuit 14 and in which a program is stored for the purpose of cleaning endoscopes of the relevant type. Such a program is for instance adapted to initially apply a high pressure to the endoscope channels, particularly intended for the removal of coarse contaminants, to subsequently apply a lower pressure for a longer period of time for the purpose of maintaining contact for a determined time between the walls of the endoscope and the cleaning liquid, and finally to apply a higher pressure, particularly for the purpose of checking whether possible blockages have been removed.

In the preferred embodiment shown in the figure pressure sensors 26A, 26B, 26C and 26D are moreover placed in each of the conduits 18A, 18B, 18C and 18D. Flow meters 27A, 27B, 27C and 27D are also arranged in each of the relevant conduits 20A, 20B, 20C and 20D. Each of the pressure sensors 26A, 26B, 26C and 26D and each of the flow meters 27A, 27B, 27C and 27D is connected to control circuit 15. It is possible to replace the individual flow meters 27A, 27B, 27C and 27D with a shared flow meter, in particular a flow meter provided with a measuring vessel.

The presence of these meters makes it possible to monitor the relevant quantities in order to prevent the endoscopes being damaged or to check whether the cleaning process is being performed in the correct manner. A high pressure together with a low flow rate thus points for instance to the presence of a blockage in an endoscope channel. It is also possible to measure and store in a memory a pressure-flow characteristic of an endoscope channel. Assuming that a characteristic of a fully cleaned endoscope channel is available, it is hereby possible to check whether such a channel is blocked and whether contaminants impeding the flow are present.

Another important function of the flow meters and pressure gauges is to monitor the correct connection of the hoses onto the channels. This is because, if a hose is loose, the cleaning liquid will flow away without much resistance. As the flow rate builds up hardly any pressure will therefore be encountered, and this is detected by the pressure gauges and flow meters. This is likewise the case for a blockage in the hose or connection, since no or hardly any flow rate will occur there as pressure builds up. A similar consideration applies when a hose is fitted to the wrong connection. This is prevented in many cases by a colour coding or by the fact that the connections fit only onto a single channel, although measurement of the pressure-flow characteristics can in many cases form an indication of a correct or incorrect connection.

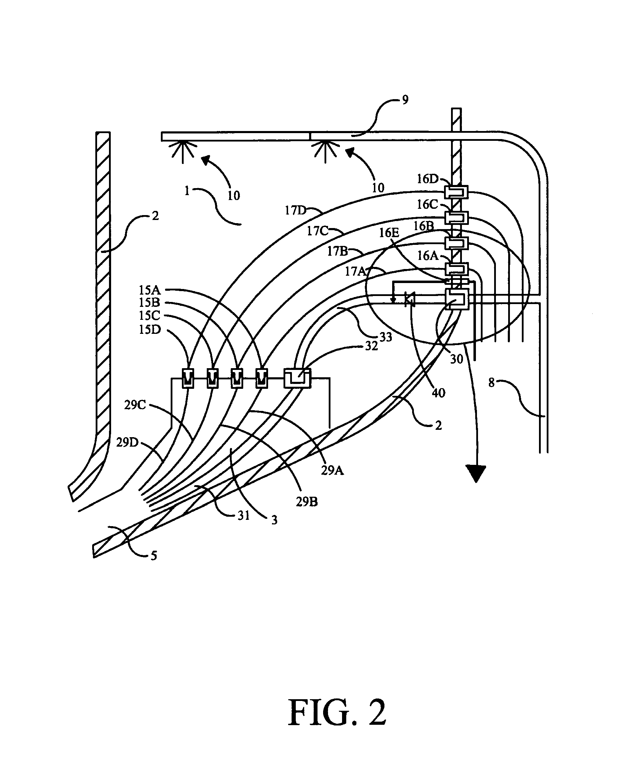

Figure 2 is a schematic detail view of a variant of the embodiment shown in figure 1. Because of the larger scale the endoscope channels 29A, 29B, 29C and 29D are shown in this figure for the purpose of elucidation. These channels connect to respective connections 15A, 15B, 15C and 15D.

The configuration shown in this embodiment differs from the configuration shown in figure 1 in that sprayers 10 are received in a fixed, i.e. non-movable, arm.

The most important difference between this embodiment and the previous embodiment lies in the presence of an additional connection 30 which is connected to the discharge channel 8 of the 'large' pump 8. This connection is thus adapted to produce a greater pressure and a higher flow rate, so that this connection is particularly suitable for endoscope channels of a large diameter. Many endoscopes are after all provided with a biopsy channel or a channel for passage of instruments, and this channel must have a greater diameter than the other channels which are only dimensioned for passage of liquid or gases or for passage of an optical fibre. The endoscope 3 shown in this figure therefore comprises such a channel 31 of a larger diameter than each of the remaining channels, and a connection 32 for said channel 31. For connection between connections 32 and 30 use is made of a connecting hose 33 with a larger diameter than that of respective connecting hoses 17A, 17B, 17C and 17D. The connections also have a larger passage area.

Because cleaning of such a channel with a larger diameter requires a higher flow rate, it can be attractive, also subject to the type of pump applied for the normal channels, to make use of another type of pump. It is then attractive to make use of the pump already present in the device, such as pump 7 used to drive the sprayers. For this purpose feed conduit 8 is provided with a branch leading to connection 30. Such pumps, which are adapted to generate a higher flow rate, are usually of the centrifugal type and do not form a closure when non-operational. In order to nevertheless prevent backflow of the cleaning liquid, a non-return valve 40 is arranged in connecting hose 33. Nor do these pumps have the property of developing pressure at a lower flow rate. Measuring a pressure-flow characteristic is therefore hardly possible in the case of such pumps.

In order to also enable measurement of these data in endoscope channels with a large passage area, a pump 19E (not shown in the figure) of the type used to supply cleaning liquid to channels forming part of the group is therefore connected in parallel to the 'large' pump 7. This pump 19E is provided in a manner similar to pumps 19A-19D with a flow meter 27E and a pressure gauge 26E. It is also driven by an electric motor 22E which is controlled by control circuit 14 via a power supply circuit 23E. For an independent control of the liquid flow to the sprayers and to the connecting piece, controllable valves can be arranged in the branch conduit or in the part of feed conduit 8 lying downstream of the branch.

## Claims

1. Device for cleaning endoscopes (3) comprising a group of several endoscope channels, comprising:
- positioning means (1,4) for placing the endoscope (3) to be cleaned;
- a reservoir (4) for cleaning liquid;
- supply means (15, 16, 17, 18, 19, 20) for supplying cleaning liquid from the reservoir (4) to the endoscope channels, forming part of the group, of an endoscope (3) to be cleaned placed in the positioning means (1,4);
- discharge means (4) for discharging to the reservoir (4) the cleaning liquid leaving the endoscope (3) to be cleaned;
- wherein for each of the endoscope channels to be cleaned and forming part of the group the supply means comprise a channel (14, 20, 18, 17) which leads from the reservoir (4) to a connecting piece (15) adapted for connection to the endoscope channels forming part of the group, and a pump (19) is placed in each of the channels (14, 20, 18, 17);
- wherein the pumps (19) are all of a type which, when non-operational, forms a closure of the channel;
- a pressure gauge (26) placed on the pressure side of the at least two pumps (19); and
- a flow meter (27) placed on the pressure or suction side of the pump (19),
**characterized in that** at least two of the pumps (19) are each coupled in each case to a controllable motor (22), that the device comprises a control element (14) which is adapted to control the motor (22) of at least two of the pumps (19),that the control element (14) is adapted to continuously regulate the flow rate of the at least two pumps (19) and that the control device (14) is adapted to measure the pressure-flow
characteristic of the endoscope channel connected to the relevant pump.

2. Device as claimed in claim 1, **characterized in that** the control element (14) comprises a memory for storing the measured pressure-flow characteristic.

3. Device as claimed in claim 1 or 2, **characterized in that** each of the pumps (19) is coupled in each case to a controllable motor (22), and that the control element (14) is adapted to control each of the motors (22).

4. Device as claimed in claim 1, 2 or 3, **characterized in that** the motors are formed by electric motors (22), and that the control element (14) is adapted to control the electric motors.

5. Device as claimed in any of the foregoing claims, **characterized in that** the control element (14) is adapted to cause at least one of the pumps (19) to operate successively at different flow rates.

6. Device as claimed in any of the preceding claims, **characterized in that** the control element (14) is adapted to cause the pumps (19) to operate initially at a high flow rate and subsequently at a low flow rate.

7. Device as claimed in any of the foregoing claims, **characterized in that** the pumps (19) are adapted to generate a flow rate lower than a predetermined flow rate when a connection is not connected to an endoscope channel.

8. Device as claimed in any of the foregoing claims, **characterized in that** the at least two pumps (19) are each formed by a displacement pump.

9. Device as claimed in claim 8, **characterized in that** the displacement pumps are formed by peristaltic pumps (19).

10. Device as claimed in any of the foregoing claims, **characterized in that** the device comprises a large pump (7) which is adapted to generate a higher flow rate than any of the other pumps (19), and that this large pump (7) is adapted for connection to an endoscope channel with a larger passage area than the endoscope channels forming part of the group.

11. Device as claimed in claim 10, **characterized in that** the large pump (7) is connected by means of a non-return valve (40) to the connection of the endoscope (3), and that there is connected between the connection of the endoscope and the non-return valve (40) a pump of the type which is adapted for connection to channels forming part of the group.

12. Device as claimed in claim 10 or 11, **characterized in that** the pump (7) with a higher flow rate is not only adapted for connection to an endoscope channel but also to a sprayer (10) for spraying the endoscope (3) placed in the positioning means.

13. Method for cleaning an endoscope (3) having a plurality of endoscope channels using a device according to claim 1, comprising the steps of:
- positioning the endoscope (3) for cleaning in a cleaning device using positioning means;
- connecting feed channels connected to pumps onto the endoscope channels of the endoscope (3) to be cleaned;
- flushing the endoscope channels at first pump powers; and
- flushing the endoscope channels at second pump powers differing from the first, wherein the first pump powers are different in different pumps and/or the second pump powers are different in different pumps,
**characterized in that** the pressure-flow characteristic of the endoscope channel is measured.

14. Method as claimed in claim 13, **characterized in that** the pressure-flow characteristic for each of the channels is stored with an identification of the endoscope (3).

15. Method as claimed in claim 14, **characterized in that** after a subsequent cleaning of the same endoscope (3) the pressure-flow characteristic is once again measured and that the measured pressure-flow characteristic is compared to the stored characteristic.

16. Method as claimed in claim 13, 14 or 15, **characterized in that** the second power is lower than the first power.

17. Method as claimed in claim 16, **characterized in that** after flushing of an endoscope channel at a high pump power a detection takes place as to whether the endoscope channel is blocked, and said endoscope channel is flushed at a low power only if it is found that the channel is not blocked.

18. Method as claimed in claim 16 or 17, **characterized in that** after flushing at the second power the endoscope channels are flushed at a third power greater than the second power.

## Patentansprüche

1. Vorrichtung (3) für das Reinigen von Endoskopen, die je eine Gruppe von mehreren Endoskopkanälen umfassen, welcher Vorrichtung umfasst:
- Positionierungsmittel (1, 4) zum Anordnen des zu reinigenden Endoskops (3);
- einen Behälter (4) für Reinigungsflüssigkeit;
- Zufuhrmittel (15, 16, 17, 18, 19, 20) zur Zufuhr von Reinigungsflüssigkeit aus dem Behälter (4) zu den Endoskopkanälen, die Teil der Gruppe bilden von einem zu reinigenden Endoskop (3), das in den Positionierungsmitteln (1, 4) angeordnet ist;
- Abfuhrmittel (4) zum Abführen der das Endoskop (3) verlassenden Reinigungsflüssigkeit in den Behälter (4);
- worin für jeden der zu reinigenden und zu der Gruppe gehörenden Endoskopkanäle, die Anschlussmittel einen Kanal (14, 20, 18, 17) umfassen, der von dem Behälter (4) zu einem Verbindungsstück (15) führt, das für die Verbindung mit den Endoskopkanälen ausgelegt ist und zu der Gruppe gehört, und worin eine Pumpe (19) in jedem der Kanäle (14, 20, 18, 17) angeordnet ist;
- worin die Pumpen (19) alle von demselben Typ sind, der im nicht operativen Zustand einen Verschluss für den Kanal bildet;
- ein Druckmessgerät (26), das sich an der Druckseite der mindestens zwei Pumpen (19) befindet; sowie
- ein Durchflussmessgerät (27), das sich an der Druck- oder Saugseite der Pumpe (19) befindet,
**dadurch gekennzeichnet, dass** zumindest zwei der Pumpen (19) jeweils mit einem regelbaren Motor (22) gekoppelt sind, dass die Vorrichtung ein Steuerelement (14) umfasst, welches für die Steuerung des Motors (22) von mindestens zwei der Pumpen (19) ausgelegt ist, dass das Kontrollelement (14) zur ständigen Steuerung der Durchfluss der mindestens zwei Pumpen (19) ausgelegt ist und dass das Steuergerät (14) zur Messung der Strömungsdruckcharakteristik des Endoskopkanals, der mit der entsprechenden Pumpe verbunden ist, ausgelegt ist.

2. Vorrichtung nach Anspruch 1, **dadurch gekennzeichnet, dass** das Kontrollelement (14) einen Speicher zum Speichern der gemessenen Strömungsdruckcharakteristik umfasst.

3. Vorrichtung nach Anspruch 1 oder 2, **dadurch gekennzeichnet, dass** jede einzelne Pumpe (19) jeweils mit einem regelbaren Motor (22) gekoppelt ist und dass das Steuerelement (14) zur Steuerung eines jeden Motors (22) ausgelegt ist.

4. Vorrichtung nach Anspruch 1, 2 oder 3, **dadurch gekennzeichnet, dass** die Motoren (22) von Elektromotoren (22) gebildet werden und dass das Steuerelement (14) zur Steuerung der Elektromotoren ausgelegt ist.

5. Vorrichtung nach einem der vorhergehenden Ansprüche, **dadurch gekennzeichnet, dass** das Steuerelement (14) ausgelegt ist, zumindest eine der Pumpen (19) nacheinander bei unterschiedlichen Durchflüssen zu betreiben.

6. Vorrichtung nach einem der vorhergehenden Ansprüche, **dadurch gekennzeichnet, dass** das Steuerelement (14) ausgelegt ist, die Pumpen (19) anfangs bei einem hohen Durchfluss zu betreiben und anschließend bei einem niedrigen Durchfluss.

7. Vorrichtung nach einem der vorhergehenden Ansprüche, **dadurch gekennzeichnet, dass** die Pumpen (19) ausgelegt sind, ein geringerer als eine vorgegebener Durchfluss zu erzeugen, wenn ein Anschluss nicht mit einem Endoskopkanal verbunden ist.

8. Vorrichtung nach einem der vorhergehenden Ansprüche, **dadurch gekennzeichnet, dass** mindestens zwei Pumpen (19) jeweils von einer Verdrängerpumpe gebildet werden.

9. Vorrichtung nach Anspruch 8, **dadurch gekennzeichnet, dass** die Verdrängerpumpen von peristaltischen Pumpen (19) gebildet werden.

10. Vorrichtung nach einem der vorhergehenden Ansprüche, **dadurch gekennzeichnet, dass** die Vorrichtung eine große Pumpe (7) umfasst, die ausgelegt ist, ein größerer Durchfluss zu erzeugen als jede andere Pumpe (19), und dass diese große Pumpe (7) für die Verbindung mit einem Endoskopkanal mit einem größeren Durchgangsbereich ausgelegt ist als die Endoskopkanäle, die Teil der Gruppe bilden.

11. Vorrichtung nach Anspruch 10, **dadurch gekennzeichnet, dass** die große Pumpe (7) über ein Rückschlagventil (40) mit der Verbindung des Endoskops (3) verbunden ist, und dass zwischen der Verbindung des Endoskops mit dem Rückschlagventil (40) eine Pumpe von demselben Typ verbunden ist, der für die Verbindung der Kanäle, die Teil der Gruppe bilden, ausgelegt ist.

12. Vorrichtung nach Anspruch 10 oder 11, **dadurch gekennzeichnet, dass** die Pumpe (7) mit einem höheren Durchfluss nicht nur für die Verbindung mit einem Endoskopkanal ausgelegt ist, sondern auch mit einer Sprüheinrichtung (10) zum Besprühen des in den Positionierungsmitteln befindlichen Endoskops (3).

13. Verfahren zum Reinigen eines Endoskops (3), das eine Vielzahl von Endoskopkanälen besitzt, unter Verwendung einer Vorrichtung gemäß Anspruch 1, die die folgenden Schritte umfasst:
- Positionieren des zu reinigenden Endoskops (3) in ein Reinigungsgerät unter Verwendung von Positionierungsmitteln;
- Verbinden von mit Pumpen verbundenen Zufuhrkanälen mit den Endoskopkanälen des zu reinigenden Endoskops (3);
- Spülen der Endoskopkanäle bei einer ersten Pumpenleistung; und
- Spülen der Endoskopkanäle bei einer zweiten Pumpenleistung, die sich von der ersten Pumpenleistung unterscheidet, worin die erste Pumpenleistung in verschiedenen Pumpen unterschiedlich ist und/oder die zweite Pumpenleistung in unterschiedlichen Pumpen verschieden ist,
**dadurch gekennzeichnet, dass** die Strömungsdruckcharakteristik des Endoskopkanals gemessen wird.

14. Verfahren nach Anspruch 13, **dadurch gekennzeichnet, dass** die Strömungsdruckcharakteristik für jeden Kanal mit einer Identifikation des Endoskops (3) abgespeichert wird.

15. Verfahren nach Anspruch 14, **dadurch gekennzeichnet, dass** nach einer nachfolgenden Reinigung desselben Endoskops (3) die Strömungsdruckcharakteristik erneut gemessen wird und dass die gemessene Strömungsdruckcharakteristik mit der abgespeicherten Charakteristik verglichen wird.

16. Verfahren nach Anspruch 13, 14 oder 15, **dadurch gekennzeichnet, dass** die zweite Leistung niedriger als die erste Leistung ist.

17. Verfahren nach Anspruch 16, **dadurch gekennzeichnet, dass** nach dem Spülen eines Endoskopkanals bei einer hohen Pumpenleistung eine Erkennung stattfindet, ob der Endoskopkanal blockiert ist, und besagter Endoskopkanal wird bei einer geringeren Leistung nur gespült, wenn sich herausstellt, dass der Kanal nicht blockiert ist.

18. Verfahren nach Anspruch 16 oder 17, **dadurch gekennzeichnet, dass** nach dem Spülen bei der zweiten Leistung die Endoskopkanäle bei einer dritten Leistung gespült werden, die größer als die zweite Leistung ist.

## Revendications

1. Dispositif pour nettoyer des endoscopes (3) comprenant un groupe de plusieurs canaux endoscopiques, comprenant :
- des moyens de positionnement (1, 4) pour placer l'endoscope (3) à nettoyer ;
- un réservoir (4) pour le liquide de nettoyage ;
- des moyens d'amenée (15, 16, 17, 18, 19, 20) pour amener du liquide de nettoyage du réservoir (4) aux canaux endoscopiques, faisant partie du groupe, d'un endoscope (3) à nettoyer placé dans les moyens de positionnement (1, 4) ;
- des moyens d'évacuation (4) pour évacuer jusqu'au réservoir (4) le liquide de nettoyage quittant l'endoscope (3) à nettoyer ;
- étant entendu que, pour chacun des canaux endoscopiques à nettoyer et faisant partie du groupe, les moyens d'amenée comprennent un canal (14, 20, 18, 17) qui va du réservoir (4) à une pièce de raccord (15) adaptée pour être raccordée aux canaux endoscopiques faisant partie du groupe, et qu'une pompe (19) est placée dans chacun des canaux (14, 20, 18, 17) ;
- étant entendu que les pompes (19) sont toutes du type qui, quand elles ne fonctionnent pas, constituent une obturation du canal ;
- un manomètre (26) placé du côté refoulement des au moins deux pompes (19), et
- un débitmètre (27) placé du côté refoulement ou admission de la pompe (19),
**caractérisé en ce qu'**au moins deux des pompes (19) sont chacune couplées dans chaque cas à un moteur régulable (22), **en ce que** le dispositif comprend un élément de commande (14) qui est adapté pour commander le moteur (22) d'au moins deux des pompes (19), **en ce que** l'élément de commande (14) est adapté pour réguler continuellement le débit des au moins deux pompes (19) et **en ce que** le dispositif de commande (14) est adapté pour mesurer la caractéristique pression-débit du canal endoscopique raccordé à la pompe correspondante.

2. Dispositif selon la revendication 1, **caractérisé en ce que** l'élément de commande (14) comprend une mémoire pour enregistrer la caractéristique pression-débit mesurée.

3. Dispositif selon la revendication 1 ou 2, **caractérisé en ce que** chacune des pompes (19) est couplée dans chaque cas à un moteur régulable (22) et **en ce que** l'élément de commande (14) est adapté pour commander chacun des moteurs (22).

4. Dispositif selon la revendication 1, 2 ou 3, **caractérisé en ce que** les moteurs sont constitués par des moteurs électriques (22) et **en ce que** l'élément de commande (14) est adapté pour commander les moteurs électriques.

5. Dispositif selon l'une quelconque des revendications précédentes, **caractérisé en ce que** l'élément de commande (14) est adapté pour amener au moins l'une des pompes (19) à fonctionner successivement à des débits différents.

6. Dispositif selon l'une quelconque des revendications précédentes, **caractérisé en ce que** l'élément de commande (14) est adapté pour amener les pompes (19) à fonctionner initialement à un débit élevé et ensuite, à un faible débit.

7. Dispositif selon l'une quelconque des revendications précédentes, **caractérisé en ce que** les pompes (19) sont adaptées pour produire un débit inférieur à un débit prédéterminé lorsqu'un raccord n'est pas raccordé à un canal endoscopique.

8. Dispositif selon l'une quelconque des revendications précédentes, **caractérisé en ce que** les au moins deux pompes (19) sont chacune constituées par une pompe volumétrique.

9. Dispositif selon la revendication 8, **caractérisé en ce que** les pompes volumétriques sont constituées par des pompes péristaltiques (19).

10. Dispositif selon l'une quelconque des revendications précédentes, **caractérisé en ce que** le dispositif comprend une grosse pompe (7) qui est adaptée pour produire un débit plus élevé que l'une quelconque des autres pompes (19) et **en ce que** cette grosse pompe (7) est adaptée pour être raccordée à un canal endoscopique ayant une section de passage plus grande que les canaux endoscopiques faisant partie du groupe.

11. Dispositif selon la revendication 10, **caractérisé en ce que** la grosse pompe (7) est raccordée au moyen d'un clapet antiretour (40) au raccord de l'endoscope (3) et **en ce que** l'on monte entre le raccord de l'endoscope et le clapet antiretour (40) une pompe du type qui est adapté pour être raccordé à des canaux faisant partie du groupe.

12. Dispositif selon la revendication 10 ou 11, **caractérisé en ce que** la pompe (7) au débit plus élevé est adaptée non seulement pour être raccordée à un canal endoscopique, mais aussi à un dispositif d'aspersion (10) pour asperger l'endoscope (3) placé dans les moyens de positionnement.

13. Procédé pour nettoyer un endoscope (3) comportant une pluralité de canaux endoscopiques en utilisant un dispositif selon la revendication 1, comprenant les étapes consistant :
- à positionner les endoscopes (3) à nettoyer dans un dispositif de nettoyage en utilisant les moyens de positionnement ;
- à brancher les canaux d'alimentation raccordés aux pompes sur les canaux endoscopiques de l'endoscope (3) à nettoyer ;
- à rincer les canaux endoscopiques à des premières puissances de pompage, et
- à rincer les canaux endoscopiques à des deuxièmes puissances de pompage différant des premières, étant entendu que les premières puissances de pompage sont différentes d'une pompe à l'autre et/ou que les deuxièmes puissances de pompage sont différentes d'une pompe à l'autre,
**caractérisé en ce que** l'on mesure la caractéristique pression-débit du canal endoscopique.

14. Procédé selon la revendication 13, **caractérisé en ce que** la caractéristique pression-débit de chacun des canaux est enregistrée avec une identification de l' endoscope (3).

15. Procédé selon la revendication 14, **caractérisé en ce que**, après un nettoyage ultérieur du même endoscope (3), l'on mesure à nouveau la caractéristique pression-débit et **en ce que** l'on compare la caractéristique pression-débit mesurée à la caractéristique enregistrée.

16. Procédé selon la revendication 13, 14 ou 15, **caractérisé en ce que** la deuxième puissance est inférieure à la première puissance.

17. Procédé selon la revendication 16, **caractérisé en ce que**, après le rinçage d'un canal endoscopique à une puissance de pompage élevée, une détection a lieu quant au fait de savoir si le canal endoscopique est obstrué et **en ce que** ledit canal endoscopique est rincé à une faible puissance seulement s'il est constaté que le canal n'est pas obstrué.

18. Procédé selon la revendication 16 ou 17, **caractérisé en ce que**, après le rinçage à la deuxième puissance, les canaux endoscopiques sont rincés à une troisième puissance supérieure à la deuxième puissance.
